(19) Europäisches Patentamt / European Patent Office / Office européen des brevets

(11) **EP 2 499 585 B1**

(12) **EUROPEAN PATENT SPECIFICATION**

(45) Date of publication and mention of the grant of the patent:
**12.06.2019 Bulletin 2019/24**

(51) Int Cl.:
*G16H 50/50* (2018.01)    *G06K 9/00* (2006.01)
*A61B 5/04* (2006.01)

(21) Application number: **10782572.1**

(22) Date of filing: **10.11.2010**

(86) International application number:
**PCT/EP2010/067216**

(87) International publication number:
**WO 2011/058059 (19.05.2011 Gazette 2011/20)**

(54) **METHODS AND SYSTEMS FOR CHANNEL SELECTION**

VERFAHREN UND SYSTEME ZUR KANALAUSWAHL

PROCÉDÉS ET SYSTÈMES DE SÉLECTION DE CHAÎNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.11.2009 GB 0919739**

(43) Date of publication of application:
**19.09.2012 Bulletin 2012/38**

(73) Proprietor: **Universiteit Gent**
**9000 Gent (BE)**

(72) Inventors:
- **CREVECOEUR, Guillaume**
  **B-9050 Ledeberg (BE)**
- **DUPRE, Luc**
  **B-8340 Sijsele (BE)**
- **HALLEZ, Hans**
  **8000 Brugge (BE)**
- **STAELENS, Steven**
  **B-9820 Merelbeke (BE)**

(74) Representative: **Wauters, Davy Erik Angelo**
**DenK iP bvba**
**Leuvensesteenweg 203**
**3190 Boortmeerbeek (BE)**

(56) References cited:
- **YITEMBE BR ET AL: "EEG inverse problem solution with minimal influence of the conductivity", COMPUTATION OF ELECTROMAGNETIC FIELDS, 17TH CONFERENCE, PROCEEDINGS, 22 November 2009 (2009-11-22), - 26 November 2009 (2009-11-26), pages 989-990, XP007917407,**
- **CHEN F ET AL: "Dipole Estimation Errors Due to Skull Conductivity Perturbations: Simulation Study in Spherical Head Models", NONINVASIVE FUNCTIONAL SOURCE IMAGING OF THE BRAIN AND HEART AND THE I NTERNATIONAL CONFERENCE ON FUNCTIONAL BIOMEDICAL IMAGING, 2007. NFSI-I CFBI 2007. JOINT MEETING OF THE 6TH INTERNATIONAL SYMPOSIUM ON, IEEE, PI, 1 October 2007 (2007-10-01), pages 86-89, XP031164095, ISBN: 978-1-4244-0948-8**
- **NEVZAT G GEN CR CER ET AL: "Sensitivity of EEG and MEG measurements to tissue conductivity", PHYSICS IN MEDICINE AND BIOLOGY, TAYLOR AND FRANCIS LTD. LONDON, GB, vol. 49, no. 5, 7 March 2004 (2004-03-07), pages 701-717, XP020024025, ISSN: 0031-9155, DOI: DOI:10.1088/0031-9155/49/5/004**
- **VANRUMSTE B ET AL: "Dipole location errors in electroencephalogram source analysis due to volume conductor model errors", MEDICAL & BIOLOGICAL ENGINEERING & COMPUTING, SPRINGER, BERLIN, DE, vol. 38, no. 5, 1 September 2000 (2000-09-01), pages 528-534, XP019834421, ISSN: 1741-0444**

- VATTA F ET AL: "Improving Lesion Conductivity Estimate by Means of EEG Source Localization Sensitivity to Model Parameter", JOURNAL OF CLINICAL NEUROPHYSIOLOGY, vol. 19, no. 1, 2002, pages 1-15, XP007917408,

## Description

### Field of the invention

The invention relates to the field of biomedical engineering. More particularly, the present invention relates to methods and systems for assisting or performing identification of electrical activity, e.g. for performing channel selection in inverse problems for the identification of electrical activity in a living creature.

### Background of the invention

Non-invasive biomedical sensors record, usually on the body surface, (multiple) signal channels related to internal changes in the human body and this usually for several time instances. For example, the electroencephalogram (EEG) measures electrical voltage signals on the scalp which result from the electrical activity inside the head. Other common examples of biomedical sensors are the magnetoencephalogram (MEG), electrocardiogram (ECG) and the magnetocardiogram (MCG). Based on the sensed signals, obtained through measurements using different sensors, also referred to as different channels, the position and type of source of electrical activity can be determined.

Starting from different channel measurements, a so-called "inverse problem" can be solved that identifies the unknown sources of the measured activity. Since the accuracy of the inverse solution is determined by the accuracy of a forward model used in the algorithm for solving the inverse problem, the forward model has to simulate the values of the biomedical channels (e.g. EEG potential values at the several measurement channels with given head geometry, source distribution and uncertain tissue) in the most accurate way. In order to obtain a useful interpretation from encephalograms or electrocardiograms, the spatial resolution of the identified electrical activity advantageously needs to be as high as possible. The spatial resolution of these techniques is amongst others determined by the number and location of sensors that is positioned on the living creature. Good spatial resolution is of importance for example when these results are used for pre-surgical evaluation of a patient suffering from neurological disorders, such as for example patients suffering from epilepsy.

Often a trade off is to be made between the required processing time, amongst others determined by the number of channels used, and the spatial resolution that is to be reached. It is known, e.g. from WO 2006/060727 to only use a subset of channels and to introduce information from the other channels in the form of synthetic data. US 2008/0161714 A1 describes a method for reducing the number of channels to be used by creating a virtual set of channels from the available channel information. The virtual set of channels thereby has less channels than the original set of channels, resulting in a reduction of processing power needed, while still providing sufficient spatial information.

Spatial resolution also is hampered by uncertainties present in the forward models used for solving the inverse problem. In the case of the EEG, MEG, ECG, and MCG, these uncertainties are typically introduced by the tissue conductivity values used since these are difficult to estimate. These uncertainties thus introduce errors in the inverse solutions, errors that can be much larger than the ones introduced by measurement noise for example.

### Summary of the invention

It is an object of the present invention that good methods and systems are provided for deriving information of internal changes of the body of a living creature.

It is an advantage of embodiments of the present invention that good, e.g. enhanced, spatial resolution can be obtained for biomedical imaging techniques based on an inverse problem. It is an advantage of embodiments according to the present invention that the accuracy of the obtained results can be good.

It is an advantage of embodiments according to the present invention that errors introduced by forward modeling uncertainties can be small or reduced.

It is an advantage of embodiments according to the present invention that errors introduced by forward modeling uncertainties can be small or reduced for uncertainties that have an impact on the measurement channel outputs where some channel outputs can be highly and others not highly sensitive to the uncertainties. Such measurement channel outputs may be the simulated results obtained in the forward numerical model and the uncertainties may be at least the effects of a change or error in the conductivity in the forward numerical model on the obtained simulated results.

It is an advantage of embodiments according to the present invention that uncertainties of the material properties (i.e. electrical conductivity values) of a living creature and/or that uncertainties of the geometrical modeling and/or that uncertainties of the placement or location of the measurement channels, can be small or reduced. The latter results in a good or improved spatial resolution of inverse problems, such as for example inverse EEG problems.

It is an advantage of embodiments according to the present invention that the influence of uncertain electrical conductivities on encephalogram or electrocardiogram inverse problem results can be reduced by channel selection.

It is an advantage of embodiments according to the present invention that selection of the channels to be used can be performed adaptively during determination of the information.

It is an advantage of embodiments according to the present invention that the methods and systems take into account different physiology of different living creatures, i.e. that methods and systems allow obtaining good accuracy substantially independent of the physiology of the living creature for whom an electrical source is characterized, e.g. a neural source or cardial source.

It is an advantage of embodiments according to the present invention that e.g. spatial position errors introduced by forward modeling uncertainties can be reduced.

The above objective is accomplished by a method and device according to the present invention.

The present invention relates to a system for estimating a property of a neural or cardial source in a subject, using inverse problem solving including a forward numerical model, the forward numerical model being used for simulating electrode potentials using lead field calculations, the model being based on geometric information of the subject, the model being based on an estimated initial property of the neural or cardial source and the model being based on a conductivity of a modeled tissue of the subject, the system comprising a receiving means adapted for receiving a plurality of measured measurement channel results from a part of the body of the subject, the measured measurement channel results being a plurality of signals responsive to the electrical activity of the neural or cardial source, a selection means adapted for selecting at least one subset of a plurality of measurement channels, said selecting comprising selecting measurement channels having a sensitivity to uncertainty in conductivity less than a predetermined value, where the sensitivity of a measurement channel being a measure of how simulated measurement channel results obtained by the forward numerical model change due to a change of uncertainty in conductivity in the forward numerical model, and a calculation means adapted for determining the property of the neural or cardial source based on the selected subset of measurement channels, the calculation means comprising: a) a forward modeling means adapted for obtaining simulated measurement channel results for the selected subset of measurement channels by the forward numerical model based on an estimated property of the neural or cardial source; b) a comparator adapted for comparing the obtained simulated measurement channel results and the measured measurement channel results of said at least one selected subset of measurement channels; c) a property calculator adapted for calculating an updated property estimate of the neural or cardial source if the measured and simulated measurement channel results differ by more than a predetermined value; and a controller adapted for controlling the receiving, selection and calculation means in an iterative manner with the updated property estimate until a sufficiently accurate agreement between the simulated and measured measurement channel results is obtained. It is an advantage of embodiments according to the present invention that more accurate determining of the property of the neural or cardial source can be obtained by taking into account a sensitivity to conductivity when selecting the channels to use. The property of the neural or cardial source is a location, an orientation, an amplitude or a dynamic behavior of an electrical activity of the neural or cardial source. Sensitivity of a certain channel to a certain uncertainty can be expressed as the change of a forward model channel due to a change in uncertainty when keeping all other inputs in the forward model constant. Some channels can have a large change (i.e. very sensitive) while others can have a small change (i.e. not so sensitive) in channel output, for the same change in uncertainty.

The calculation means may be adapted for determining, e.g. estimating, a location of the neural or cardial source. It is an advantage of embodiments according to the present invention that accurate determination of the location of neural or cardial sources may be performed, e.g. as input for surgery or for performing diagnostics based thereon.

It is an advantage of embodiments according to the present invention that these can especially be used when applying forward numerical modeling, resulting in more accurate determination of the property of the neural or cardial source. It is an advantage of embodiments according to the present invention that these can be used with different forward models. It is an advantage of embodiments according to the present invention that the gain in accuracy by taking into account sensitivity to conductivity can be obtained substantially independent from the forward model used, as long as this forward model includes sensitivity to conductivity.

It is an advantage of embodiments according to the present invention that conventional techniques such as for example least square minimization can be used. The calculation means is adapted for determining a new estimate of the property of the neural or cardial source based on the comparing of the expected measurement channel results and the measured measurement channel results.

The controller may be adapted for dynamically selecting a new subset of measurement channel results for subsequent iterative steps. The selection means may be adapted for furthermore taking into account the sensitivity of the measurement channel results to a further uncertainty in the measurement channels for the neural or cardial source. The further uncertainty may be any or a combination of a location of probes used for obtaining measurement channel results, a change in properties with respect to the surrounding bodily part due to a lesion or a geometric uncertainty.

The present invention also relates to a computer-implemented method for estimating a property of a neural or cardial source in a subject, using inverse problem solving including a forward numerical model, the forward numerical model being used for simulating electrode potentials using lead field calculations, the model being based on geometric information of the subject, the model being based on an estimated initial property of the neural or cardial source and the model being based on a conductivity of a modeled tissue of the subject, the property being a location, an orientation, an amplitude or a dynamic behavior of an electrical activity of a neural or cardial source, the method comprising

- receiving a plurality of measured measurement channel results from a part of the body of the subject, the measured measurement channel results being a plurality of signals responsive to the electrical activity of the neural or cardial source;

- selecting at least one subset of a plurality of measurement channels, said selecting comprising selecting measurement channels having a sensitivity to uncertainty in conductivity less than a predetermined value, the sensitivity of a measurement channel being a measure of how simulated measurement channel results obtained by the forward numerical model change due to a change of uncertainty in conductivity in the forward numerical model, and

- estimating the property of the neural or cardial source based on the selected subset of measurement channels, the estimating comprising: a) forward modeling of the simulated measurement channel results for the subset of measurement channels based on an estimated property based on an estimated property of the neural or cardial source; b) comparing the simulated measurement channel results and the measured measurement channel results of said at least one selected subset of measurement channels; c) calculating an updated property estimate of the neural or cardial source of the measured and simulated channel results differ by more than a predetermined value; and repeating the receiving, selecting and calculating in an interative manner with the updated property estimate until a sufficiently accurate agreement between the simulated and measured measurement channel results is obtained.

Estimating a property of the neural or cardial source comprises comparing the expected measurement channel results and the measured measurement channel results.

The invention also relates to a machine readable data storage device storing a computer program product for performing, when executed on a computer, a method as described above.

Particular and preferred aspects of the invention are set out in the accompanying independent and dependent claims. Features from the dependent claims may be combined with features of the independent claims and with features of other dependent claims as appropriate and not merely as explicitly set out in the claims. These and other aspects of the invention will be apparent from and elucidated with reference to the embodiment(s) described hereinafter.

**Brief description of the drawings**

FIG. 1 illustrates the difference between a forward problem and an inverse problem, the inverse problem being the type of problems that especially can benefit from embodiments according to the present invention.

FIG. 2 illustrates an example of off-line construction of an EEG forward model, as can be used in an embodiment according to the present invention.

FIG. 3 illustrates an example of a method for obtaining information regarding a neural or cardial source making use of a method for channel selection according to an embodiment of the present invention.

FIG. 4 illustrates an example of a method for localizing a neural or cardial source using channel selection according to an embodiment of the present invention.

FIG. 5 illustrates a system for localizing a neural or cardial source using channel selection according to an embodiment of the present invention.

FIG. 6 illustrates a computing device for performing a method for localizing a neural or cardial source using channel selection according to embodiments of the present invention.

FIG. 7 illustrates a simple head model used for obtaining a first set of experimental results using an embodiment of the present invention.

FIG. 8 to FIG. 12 illustrate a comparison of dipole localization errors obtained through a conventional method with the dipole localization errors obtained using a selection method according to embodiments of the present invention.

FIG. 13 illustrates indices of the selected channels in each iteration used in an exemplary selection method according to an embodiment of the present invention.

FIG. 14 shows a source localization error versus different assumed soft tissue/skull conductivity ratios, illustrating effects of embodiments of the present invention.

FIG. 15 shows a dipole position error as function of hardware setups used, illustrating effects of embodiments of the present invention.

FIG. 16 illustrates the dipole position error for the presence of two dipoles, illustrating effects of embodiments of the present invention.

FIG. 17 illustrates an axial slice of a realistic head model geometry used for performing an exemplary method according to an embodiment of the present invention.

FIG. 18 illustrates a dipole position error as function of an assumed conductivity ratio, illustrating effects of embodiments of the present invention.

FIG. 19a to FIG. 21b illustrate dipole localization errors due to using wrong conductivity ratio when using traditional methodology (a) and channel selection methodology (b) for different dipole orientations.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

## Detailed description of illustrative embodiments

Embodiments of the present invention can be applied to different types of inverse problems, such as for example electroencephalography (EEG), magnetoencephalography(MEG), electrocardiography (ECG or EKG), magnetocardiography (MCG), etc. The latter techniques provide information regarding activity, e.g. electrical activity, of a part of a living creature, such as for example of a brain or heart of human being.

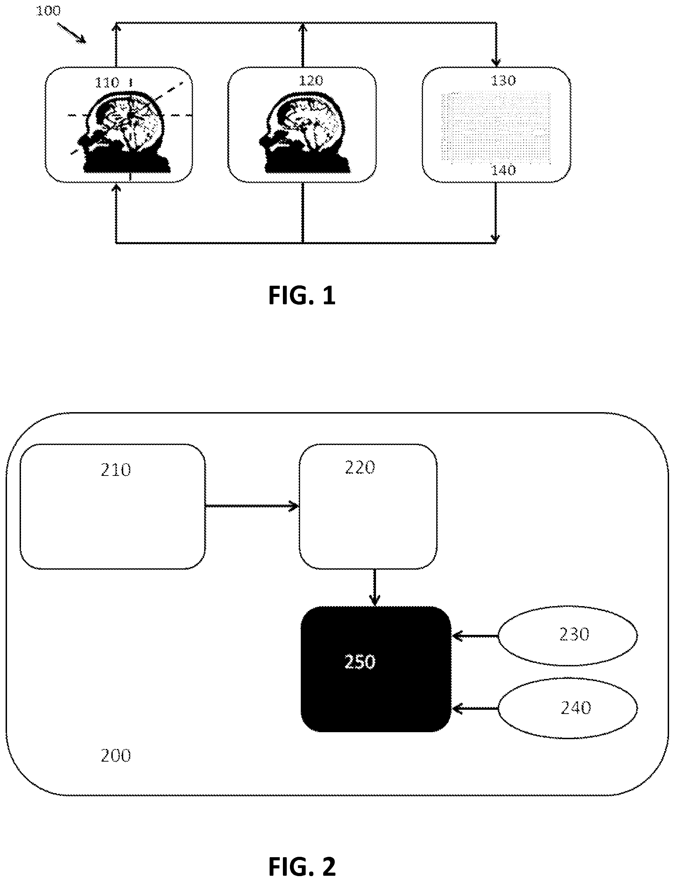

Where in embodiments of the present invention reference is made to the term inverse problem, reference is made to the situation whereby the property of an unknown source of a signal activity is determined based on measurement of signals or fields, generated by the source, at a distance from the source. Typically, the inverse problem is solved by an iterative procedure that sequentially evaluates a forward model whereby the solution is obtained when the simulated sensor signals converge to the actually measured signals. One example thereof is the localization of a source of electrical energy inside the brain by sensing signals outside the scalp. FIG. 1 illustrates both forward and inverse problems. The forward problem is illustrated by the arrow at the top, whereby starting from the source and taking into account a head model, simulations of the signal to be expected are obtained. The inverse problem is illustrated by the arrow at the bottom, whereby starting from signals measured e.g. externally, the source position is estimated or determined, taking into account a head model. FIG. 1 illustrates a method 100 indicating how based on a source model 110 and based on a head model 120, including a geometrical configuration of the head model 120 with segmentation of several tissues whereby to each tissue a conductivity is assigned, EEG simulations 130 are obtained allowing interpretation of EEG measurements 140. Embodiments according to the present invention are especially suitable for dealing with inverse problems.

Where in embodiments of the present invention reference is made to sensitivity of a channel to an uncertainty, reference is made to a measure of how a channel is influenced by an uncertainty or how the simulated measurement channel signals, e.g. potentials, can change in a particular channel due to a change of uncertainty in conductivity in the forward model. It thus may be regarded as how sensitive the result for a given measurement channel is upon a fluctuation of a parameter caused by an uncertainty.

Where in embodiments of the present invention reference is made to the conductivity, reference is made to a measure for the ability for tissue to conduct electrical currents.

Where in embodiments of the present invention reference is made to uncertainties, reference is made to the situation where values of properties are not exactly known. These thus can be regarded as uncertainties, some examples thereof being the conductivity values of tissue, geometrical properties or electrode positions. Uncertainties can be given as input to the forward modeling and influence the output of the forward modeling.

Where in embodiments of the present invention reference is made to a neural or cardial source, the latter includes a case where the source comprises a plurality of distributed sub-sources, wherein the sub-sources may for example be different dipoles positioned on a different position. Alternatively, this can be formulated as the system/method being applicable for at least one neural or cardial source or as the system/method being applicable to one or more neural or cardial sources.

According to a first aspect, embodiments of the present invention relate to a method for estimating a property, e.g. the location, of a neural or cardial source using an inverse problem. The method according to embodiments of the present invention makes use of selection of a subset of measurement channels to reduce inaccuracy in the determination of the property of the neural or cardial source. The method is especially suitable for performing electroencephalography (EEG), magnetoencephalography (MEG), electrocardiography (ECG or EKG) or magnetocardiography (MCG), although the invention is not limited thereto. According to embodiments of the present invention, the method comprises selecting a subset of a plurality of measurement channel results taking into account the sensitivity of the measurement channel results to conductivity for the neural or cardial source under study and estimating a property of the neural or cardial source based on the selected subset of measurement channel results. The method may be an iterative method, whereby the selecting and the estimation is performed iteratively to obtain a better estimation of the property or of other features following from the method (e.g. better conductivity values). In accordance with some embodiments of the present invention the obtained property of the neural or cardial source is not a diagnosis as such nor does it provide or lead to a diagnosis directly. That is, in accordance with some embodiments, the clinical parameter is only information from which relevantly trained personnel could deduce some form of diagnosis however only after an intellectual exercise that involves judgment.

By way of illustration, the present invention not being limited thereby, an exemplary method for estimating a property of a neural or cardial source is discussed, illustrating standard and optional features and advantages of embodiments according to the present invention.

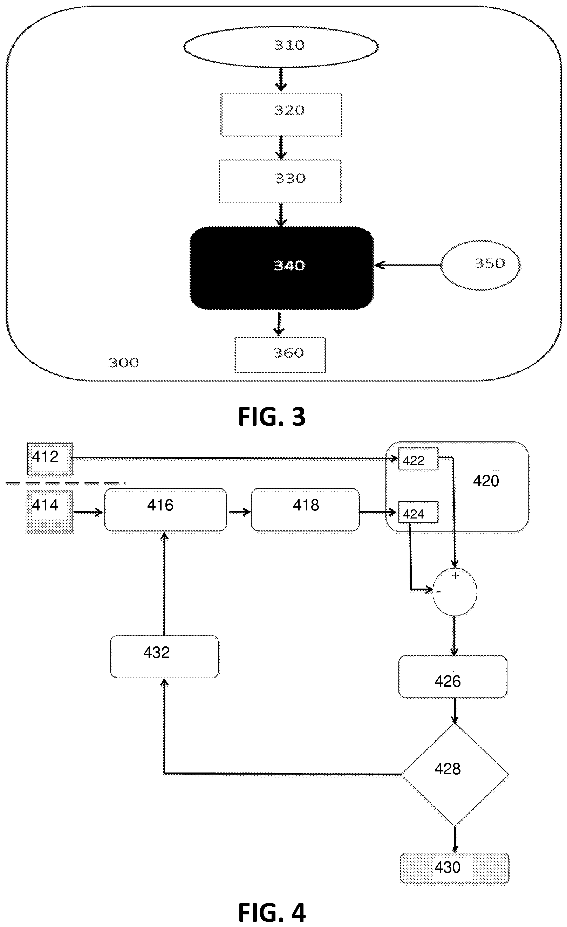

In a first step, the method may comprise receiving input, as shown in the method 300 of FIG. 3. The input 310 may comprise a plurality of measurement channel results. Such input may be received as a data set or alternatively may be obtained by sensing a plurality of signals responsive to electrical activity of the neural or cardiac source through a plurality of sensors. In other words, using a plurality of sensors, receiving input also may comprise acquiring data, in the present example being expressed as EEG acquisition. The sensing as such may be part of the method or may not be part of the method. The method advantageously makes use of a forward model and receiving input may also comprise receiving the model to be used or receiving calibration values for the model to be used. In the present example, the model is constructed off line. In one example, as illustrated in scheme 200 of FIG. 2, construction of the model may be perfomed by obtaining geometric information 210, e.g. MRI information, construction of a volume conductor model with incorporation of the geometry of the patient 220, defining the material properties, e.g. conductivity values, of several tissues 230, defining the electrode positions 240, performing lead field calculations 250 and performing an off-line construction of an EEG forward model. The model of the present example is an EEG forward model, determined based on geometric information of the patient, e.g. obtained through medical imaging such as MRI. Magnetic resonance imaging is well known by the person skilled in the art. The model thereby provides lead field calculations to allow modeling of measurement channel results for different measurement channels, e.g. electrode positions, starting from a neural or cardiac source at a position for which the input is provided. The model may be a parameter based model. Receiving input may comprise receiving input regarding an estimated initial property, e.g. position, of the neural or cardiac source and estimated conductivity values for the living creature under study. In a second step, filtering 320 may be performed on the received input. The filtering may be based on several basic techniques. Basic techniques can be based on the frequency content of the signal of interest. This is typically done by eliminating frequency bands which correspond to noise. Advanced methods, such as Blind Source Separation, try to represent the signals as a linear mixture of source signals. The source signals are imposed to some certain statistical constraints. Principal component analysis (PCA) provides a orthogonal mixture sorted according to the variance of the source signals. Independent component analysis (ICA) provides a mixture where the source signals are statistically independent. The filtering also may include artifact filtering 330. Artifacts can be generated by electrical activity from outside the heart or brain. Examples of such artifacts are muscle activity, eye blinks, respiratory activity,.... These artifacts can distort the measured signals and thus also the automated interpretation of these. Mostly artifacts are removed using Blind Source Separation techniques or by rejecting the channel where the artifact is present.

In a third step, the method comprises selecting a subset of measurement channels of the neural or cardial source 340. The channels thereby are selected such that the selection takes into account sensitivity to conductivity for the measurement channel. The sensitivity may depend on the reliability of the conductivity values used. Conductivity of biological tissue is referred here as the material's ability to conduct an electrical current. The conductivity of biological tissue can in a general way be represented by a 3-dimensional matrix. Each value in this matrix represents the directionally dependent material's ability to conduct an electrical current. This matrix can represent tissue with anisotropic behavior. In the isotropic case, this matrix can be reduced to a scalar value. The conductivity values initially may be estimated from measurements, as e.g. discussed by Oostendorp et al in IEEE Transactions on Biomedical Engineering 47 (11), pp1487-1492 (2000) or by Goncalves et al. in IEEE Transactions on Biomedical Engineering 50 (6), pp754-767 (2003) or may be estimated from models, such as for example the 4-Cole-Cole model as discussed by Cole et al. in Journal of Chemical Physics 9, pp 341-351 (1941) and Gabriel et al. in Physics in Medicine and Biology 41, pp2271-2293 (1996). It is an advantage of embodiments according to the present invention that through iteration updated, i.e. more accurate, conductivity values can be obtained and can consequently be used. Selection may be performed by taking the different measurement channel results and corresponding conductivity values as an input and performing differentiation of the measurement channel results to the conductivity, evaluated for the estimated conductivity values. Selection of a subset may be performed by selecting all measurement channels results being less sensitive to conductivity than a predetermined value, by selecting a particular number of measurement channel results having the lowest sensitivity to conductivity of the plurality of results, etc. The number of channels (N) selected in the subset from the plurality of channels (M), advantageously comprises those channels that provide useful information but are less prone to conductivity uncertainties. The number of selected channels (N) thereby may be at least the number of parameters that is to be determined for the property of the neural or cardiac source to be estimated. The latter may be performed iteratively, as indicated by arrow 350.

In a following step, the method comprises determining the property 360 of the neural or cardiac source based on the selected subset of measurement channel results in the least squares sense. The neural or cardiac source representation may be application dependent and may in some examples be represented by one or a limited number of dipoles. Such determination may comprise forward modeling of the expected measurement channel results based on an estimated position of the neural or cardiac source, comparing the expected measurement channel results and the measured measurement channel results and evaluating whether or not the difference between the measured and modeled results is sufficiently small. If the latter is the case, it is decided that the property of the neural or cardiac source is sufficiently accurate. If the difference between the measured and modeled results is considered too large, a new estimated position is estimated for the neural or cardial source, and the selection and determination step are repeated using updated estimated property and optionally also updated estimated conductivity values. The new estimated position may be determined based on a predetermined algorithm, a minimization or optimization algorithm such as Nelder-Mead simplex method, stochastic minimization method (genetic algorithm, etc.), etc. A more detailed description of a flow chart expressing the determination of an estimated property and the selection of a subset of channels will be provided below. The steps may be repeated until a sufficiently accurate agreement between the modeled and measured measurement channel results is obtained. The latter may be determined by predetermined rules, such as for example a difference value that is smaller than a predetermined value or the number of iteration steps becoming too large.

Once a sufficiently accurate agreement between modeled and measured measurement channel results is obtained, the corresponding property of the neural or cardial source is outputted, either to a memory, as data output or to a display, as also shown in FIG. 3.

By way of illustration, embodiments of the present invention not being limited thereto, an example of an algorithm according to an embodiment of the present invention is illustrated by the flow chart shown in FIG. 4. The algorithm is based on channel selection implemented in an EEG inverse problem. The algorithm starts from measured EEG signals and recovers the location of the neural or cardial sources that correspond with these signals. It furthermore uses initial dipole parameters and intial conductivity values. The EEG signals in the present example are measured using a certain configuration of sensors (electrodes) that are placed on the scalp of the person under study. It is assumed that the M sensor positions are known. M signals (potentials) at a certain time instance thus are recorded when using the sensors. In the present example, a numerical forward model is able to simulate EEG potentials, providing a proper head model of the living creature under study. The head model includes a proper geometry of the living creature under study and the conductivity of the several tissues. Other uncertain conductivity values, such as conductivity of cerebrospinal fluid (CSF) can be used as additional parameter in the head model. The parameter values are difficult to determine experimentally and the uncertainty of the parameters influences to a large extend the spatial resolution of the estimated location of the neural or cardial source.

The inverse problem typically may take the following inputs :

- Measured signals 412 such as for example EEG data. For M measurement channels being available, the obtained measurements are, at a certain time instance, the M measurement results. These are indicated as $\underline{F}_{EEG}$ being an M dimensional vector.

Such a vector also can correspond to a so-called topography vector of a spatio-temporal EEG MxT matrix, being a vector expressing the topologically arranged measurement results at a certain time t. The measurement signals may be electrical signals, magnetic signals, etc., depending on the particular type of inverse problem that is solved. The measured signals may be measured using conventional sensors, such as for example, EEG/ECG electrodes (Ag/AgCl electrodes, gold electrodes), MEG sensors (multiaxial gradiometers, magnetometers), MCG sensors such as multiaxial gradiometers, magnetometers,).

- For the method, the input involves an initial estimate of the conductivity values 414, where the conductivity ratio, indicated as $\tilde{X}$, is widely used. This input $\tilde{X}$ is e.g. related to EEG and MEG. The conductivity ratio referred hereto is the ratio of soft tissue conductivity to skull conductivity. This conductivity ratio is widely used in EEG applications because the channels highly depend on this conductivity ratio. The initial estimate of the conductivity ratio may for example be based on previously measured values, on a model, etc. The conductivity typically may be a large source of inaccuracy. $\tilde{X}$ can also refer to other uncertainties such as geometrical related uncertainties, uncertainties on the measurement positioning, uncertainties of other brain tissue such as CSF, etc. $\tilde{X}$ can also comprise multiple uncertainties. For the ECG an MCG application, $\tilde{X}$ can be the cardiac tissue conductivity values.

- For the minimization method, a start value for the position of the source $\underline{r} = [x, y, z]^T$ is also used as input. Often a standard position, such as for example the centre of the object under study, e.g. the brain, is selected as the position of the source. It is also possible to use a random start value of the source.

The method iteratively uses a forward model for solving the inverse problem. Different forward models can be used. The forward model a representation of physical properties of the human head. The most common physical properties used in the representation are geometry and conductivity. To obtain the electrode potentials caused by a neural or cardial source in the forward model, Poisson's equation is solved. Traditional methods use concentric spheres or ellipsoids to model the geometry. These multi-spherical or multi-ellipsoidal models use isotropic conductivities. Although these head models are a coarse representation of reality, the advantage lies in the fast solution of Poisson's equation. Some forward models are modeled more realistic as multiple surfaces each representing the interface between tissues. Using such models, a numerical method, such as Boundary Element Method (BEM), typically is used. This method typically involves the inversion of a square matrix (~10000-50000 rows and colums), which once completed can be used to solve the forward problem in a fast way. However, these models can not incorporate anisotropic conductivities and were limited to homogeneous structures. In reality the human head is heterogeneous and several tissues have an anisotropic conductivity. Realistic volume based methods directly use the information from a anatomical medical scan. Through this technique, parts of the human head which have a reasonable influence on the forward model, such as eyes, sinuses, ventricular system,... , can be incorporated in the forward model. The solution of Poisson's equation makes use of volume based numerical techniques, such as Finite Element Method or Finite Difference Method. As these models typically may consist of 2 - 10 million elements, iterative solvers often may be required to solve Poisson's equation. Although computationally intensive, these models have proven to be very accurate. In a first step 416, the EEG potentials $F_{EEG}$ are calculated using the given numerical forward model that correspond with source location, e.g. dipole location $\underline{r}$ and uncertainty or multiple uncertainties $\tilde{X}$, resulting in EEG potentials

$$\underline{V}_{EEG} = \underline{L}(\underline{r}, \tilde{X})\underline{d} = \underline{L}(\underline{r}, \tilde{X})\underline{L}(\underline{r}, \tilde{X})^{\dagger} \underline{F}_{EEG}$$

herein $\underline{L}$ is the $M \times 3$ lead field matrix that depends on the numerical head model (geometry), the positioning of the measurement system and the conductivity values. Here a sub-optimal least squares estimator of the dipole orientation is used by the Moore-Penrose pseudoinverse,

$$\underline{d}_{opt} = \underline{L}(\underline{r}, \tilde{X})^{\dagger} \underline{F}_{EEG}$$

Other estimators also may be used. This can be extended when using multiple uncertain conductivity values (conductivity of the cerebrospinal fluid, etc.) and when using multiple neural or cardial sources. $\underline{L}$ can be determined off-line. The latter is illustrated in FIG. 2, showing that for example MRI images can be used for determination of the geometry of the patient, from which lead field calculations can be performed, using electrode positions. The measured EEG signals also are used.

In a second step, calculation is performed of the sensitivity S 418. The sensitivity of a channel to an uncertainty is a measure of how a channel can be influenced by an uncertainty or how the measured potentials can change due to a change of uncertainty. A possible means for measuring the sensitivity is by calculating_a first order derivative thereof of the EEG potentials or of the lead fields $\underline{L}$ to the conductivity ratio X :

$$S = \frac{\delta V_{EEG}}{\delta \underline{X}} \qquad \text{or} \qquad S = \frac{\delta \underline{L}}{\delta \underline{X}}$$

which is evaluated at $\underline{X} = \tilde{X}$. The sensitivity can be calculated through finite differentiation or using another numerical method. Other means of calculating the sensitivity are to calculate in a Bayesian framework the EEG potentials or the lead fields due to an uncertainty distribution. The standard deviation of the probability density function of each channel can be a measure of sensitivity. Other sensitivity estimators may be used.

Based on the sensitivity S, the potentials that have a large influence on the potential values can be selected. If a threshold $\varepsilon$ is defined, potentials can be selected which follow $S_i \langle \varepsilon, i=1,....,N$ and in this way the potentials with smallest sensitivity are selected 420. The latter can for example be performed by comparing selected measured EEG channels 422 based on the measured input and selected calculated EEG channels 424 based on the calculated sensitivity. Such a comparison may include comparison of the channel values themselves or derivatives thereof. An example thereof is to compare the selected topographies based on the measured input and the selected calculated topographies based on the calculated sensitivity. Other selection strategies also may be chosen by the user. For example, selection of a predetermined number of potentials having the lowest sensitivity to conductivity can be performed. In this way, the calculated lead fields or potentials can be selected, e.g. $\underline{V}^S_{EEG}$, and the corresponding electrodes can be selected for the measured data, i.e. $F^S_{EEG}$.

In a following step, the cost function $\Delta \underline{V}$ 426 of the EEG inverse problem is then determined as

$$\Delta \underline{V} = \cos t(\underline{V}^S_{EEG}, F^S_{EEG})$$

The cost function can be traditionally defined as the least squares difference between measured and simulated EEG data, i.e.

$$\cos t(\underline{X}, \underline{Y}) = \left\| \underline{X} - \underline{Y} \right\|^2$$

When multiple sources need to be estimated, the cost function can be represented by the Multiple Signal Classification (MUSIC) or the Recursively Applied and Projected (RAP) - MUSIC cost function, see Mosher and Leahy in IEEE Transactions on Signal Processing 47, pp 332-340 (1999). In a following step, due to the use of selected potentials, an alternative cost function needs to be defined. Due to the fact that the set of potentials that is calculated can be reformulated in the first order as :

$$\underline{V}^S_{EEG}(\underline{X}) = \underline{V}^S_{EEG} + \underline{S}^{(k)}(\underline{X} - \tilde{\underline{X}})$$

The cost function thus can be reformulated as the correlation between $\Delta \underline{V}$ and the sensitivity $\underline{S}$. Furthermore an estimate of the conductivity is obtained.

In a following step, if the termination criteria are reached, the algorithm is stopped 428. The termination criterion may be given by the user and may be determined as e.g. a cost value that is smaller than a certain tolerance value. At that moment, the correct dipole position $\underline{r}^*$ 430 is obtained. If the termination criterion has not been reached, the algorithm is continued.

In a following step, the location of the dipole 432 then is updated

$$\underline{r} = \underline{r} + \underline{h}$$

and the forward calculation of the potentials is again performed by returning the algorithm to step 1. The above steps can also be extended for recovering multiple sources by using a proper cost function and the above steps can also be executed sequentially, which is e.g. the case for the minimization of the RAP-MUSIC cost functions.

In some embodiments, as already hinted for above, besides the sensitivity to conductivity, also one or more other uncertainties can be taken into account using a method according to embodiments of the present invention and thus the effect of other uncertainties also can be small, reduced or minimized. These additional uncertainties may be any type of uncertainty whereby different channels have a different sensitivity to the uncertainty. Some examples can be change of conductivity in a lesion with respect to the surrounding bodily part, geometric uncertainties, uncertainties regarding the positioning of the electrodes, etc.

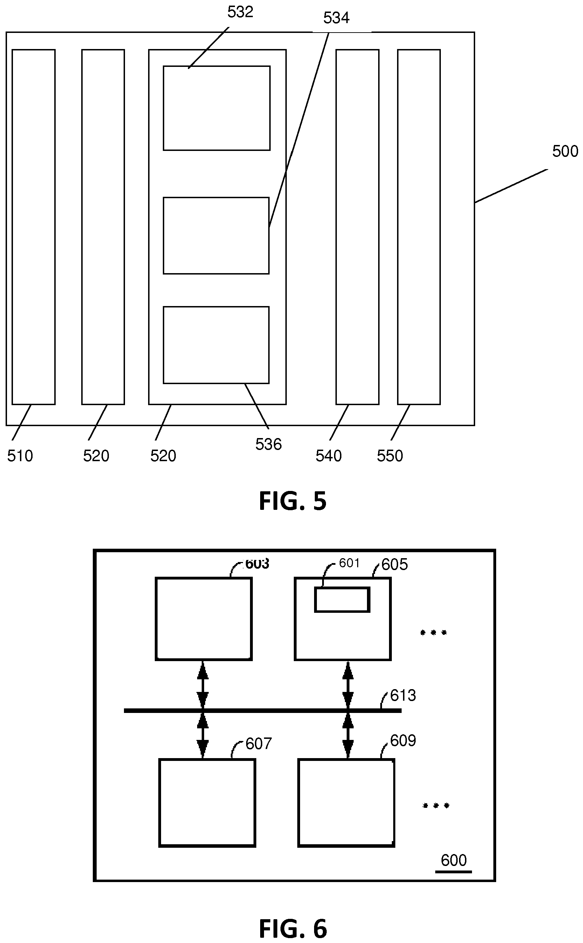

In one aspect, the present invention relates to a system for estimating a property of a neural or cardial source, e.g. in a living creature. The system may especially be suitable for determining electrical activity of a heart or a brain of a living human being, although the invention is not limited thereto. The system may be especially suitable for extracting information from electroencephalography (EEG), magnetoencephalography (MEG), electrocardiography (ECG or EKG) or magnetocardiography (MCG), although the invention is not limited thereto. The system may be a medical device or may be part of a medical device for performing encephalography or electro- or magnetocardiography. According to embodiments of the present invention, the system is adapted for estimating a property, such as position, of a neural or cardial source using inverse problem solving, whereby the system comprises a selection means for selecting at least one subset of a plurality of measurement channel results. Selecting thereby takes into account the sensitivity of the measurement channel results to conductivity for the neural or cardial source. The system also comprises a calculation means for determining a property of the neural or cardial source based on the at least one selected subset of measurement channel results. A more detailed description of an exemplary system, illustrating features and optional features of the system is further described with reference to FIG. 5.

The system 500 typically may comprise a receiving means 510 for receiving a plurality of measurement channel results from a part of the body of a living creature. Such receiving means may be an input port for receiving data results recorded earlier. The actual recording thus does not need to be part of embodiments of the present invention. Alternatively, the receiving means 510 may comprise a recording means for recording a plurality of measurement channel results. One example of a receiving means 510 may comprise a set of sensors that is adapted for obtaining a set of measurement channel results. The number of sensors present in the receiving means 510 may be selected in view of the application. The number of sensors typically may be in the range between 1 and 350 sensors, but can be easily extended. The range may vary from application to application, and may e.g. be between 1 and 256 for EEG applications, such as for example between 20 and 50 sensors e.g. when applying EEG for clinical use, for example between 128 and 256 sensors e.g. when applying EEG for experimental psychology purposes. The number of sensors may for example be up to 64 sensors when applying ECG and for example up to 350 when applying MEG. The sensors may be sensors adapted for measuring an effect of electrical activity of a neural or cardial source, such as for example electrical sensors or magnetic sensors, although the invention is not limited thereto. The different sensors result in different measurement channels. Due to the inherent variation of conductivity throughout the body of a living creature, dependent e.g. on the shape and tissue type at different locations on the body, some measurement channels will be more sensitive to conductivity than others. Embodiments of the present invention make use thereof to minimize accuracy of the determined property of the neural or cardial source.

The receiving means 510 may be adapted for receiving the plurality of measurement channel results in a topologically arranged manner. In this way, it can be known which topological position on the living creature corresponds with which measurement channel result.

The receiving means furthermore may be adapted for receiving other input, such as for example an initial position estimation of the neural or cardial source, initial conductivity values for the measurement channels, a forward model or parameters determining a forward model, etc.

The system 500, according to embodiments of the present invention, comprises a selection means 520 or selector 520 for selecting a subset of measurement channel results. The selection means 520 thereby is adapted for performing the selection taking into account sensitivity to conductivity for the measurement channel. The selector may take the different measurement channel results and corresponding conductivity values as an input and select a subset of measurement channel results as an output by performing differentiation of the measurement channel results to the conductivity. The differentiation may be performed e.g. through finite differentiation, although the invention is not limited thereto.

The system 500 also comprises a calculation means 530 for determining the property of the neural or cardial source based on the at least one selected subset of measurement channel results. The calculation means 530 therefore may comprise a forward modeling means 532 for forward modeling based on an estimated position of the neural or cardial source the expected measurement channel results for the subset of measurement channel results. The forward model applied may be in any suitable model, such as in the case of a neural source a simplified multi-spherical head models as realistic head models derived from MR and X-ray CT images. In realistic head models, the tissue types may be modeled as isotropic or anisotropic conductor. It may be determined upfront and off line, as e.g. illustrated by FIG. 2.

The calculation means 530 also may comprise a comparator 534 for comparing the expected measurement channel result and the measured measurement channel result. The comparator 534 may for example determine a cost function of the inverse problem, whereby the cost function may for example be determined by a least square difference between measured and modeled result. The cost function also may be a higher order relationship between the measured and modeled results. Such functionalities can easily be programmed, both in software and/or in hardware.

The calculation means 530 furthermore advantageously may comprise a property calculator 536 for calculating a more accurate property estimate of the neural or cardial source. The latter may be performed if for example the measured and modeled measurement channel results do not coincide or if these differ more than a predetermined value. The calculation of a more accurate property estimate may be performed using predetermined rules. The step $h$ can be updated using a predetermined optimization or minimization algorithm such as for example Nelder-Mead simplex method, genetic algorithm, etc.

The system 500 furthermore advantageously may comprise a controller 540 for controlling the selection and/or calculation means in an iterative manner such that the property of the neural or cardial source can be determined in an iterative way. Based on the determined more accurate property estimate, an iteration of the measurements and forward modeling may be performed and an evaluation of the obtained results may be performed. Whereas in some embodiments of the present invention, each time a new selection of the subset may be performed, alternatively the selected subset may be used in subsequent iteration steps.

The controller furthermore may have the functionality of controlling the receiving means 510, thus controlling the input of the system. The controller 540 may control the input data. In some embodiments, the controller 540 also may be adapted for controlling the capturing of data by controlling the sensing by the plurality of sensors. For some applications, where the location of the neural or cardial source is not changing much in time, i.e. where there is static electrical activity, the same selection can be used for each time sample. For such applications, the sub-selection made can be maintained and the step of re-selecting a sub-set of measurement channels can be omitted in an iterative process. The need for updating the sub-selection may be determined by the timescale of the variation of the measurement channel results and the variation of the neural or cardial source.

The system 500 furthermore advantageously may comprise a memory 550 for storing the results obtained, for storing the initial set of conductivity values as well as optionally updated conductivity values, an estimated initial position of the neural or cardial source and optionally for storing the measurement and/or estimated measurement channel results at least temporarily. Such a memory may be a conventional memory component, as known in the art. Other values, intermediate results or output results also may be stored shortly, temporarily or for a longer time. The system 500 furthermore may comprise an output means for outputting the calculated results.

The system 500 advantageously may be adapted, e.g. through control signals of the controller, for providing neural or cardial source property information for a given timescale. Typically neurons act produce signals in the order of 0 to 70 Hz. Hence, activity changes in the millisecond scale. To improve the signal-to-noise ratio of the measurements, a window of multiple time series can be considered assuming that the sources are stationary in that window, e.g. a epileptic spike is active during 250 ms, the start of an epileptic seizure may be stationary during the first second. Thus the presented technique can for example be performed on each time sample or on consecutive time windows of 0.5 to 1 second. In this way a dynamical evolution of the neural or cardial source(s) in the living creature can be made visible.

The system 500 furthermore may comprise components being able for generating the functionality of part of, one or more method steps as described above. Whereas the controller has been described as forming part of the system, embodiments of the present invention also relate to controllers for controlling a system as described above or to controllers for performing a method as described above.

It is an advantage of embodiments of the present invention that these enhance the spatial resolution of biomedical inverse problems, which may be highly relevant for diagnostic or surgical purposes (e.g. planning brain surgery in case of epilepsy where location precision is crucial).

The above described method embodiments for estimating a property, e.g. a position, of a neural or a cardial source may be at least partly implemented in a processing system 600 such as shown in Fig. 6. Fig. 6 shows one configuration of processing system 600 that includes at least one programmable processor 603 coupled to a memory subsystem 605 that includes at least one form of memory, e.g., RAM, ROM, and so forth. It is to be noted that the processor 603 or processors may be a general purpose, or a special purpose processor, and may be for inclusion in a device, e.g., a chip that has other components that perform other functions. Thus, one or more aspects of the present invention can be implemented in digital electronic circuitry, or in computer hardware, firmware, software, or in combinations of them. For example, the forward modelling of the expected measurement channel results of the subset and/or the selection of the subset of measurement channel results taking into account the sensitivity to conductivity may be a computer implemented step. The processing system may include a storage subsystem 607 that has at least one disk drive and/or CD-ROM drive and/or DVD drive. In some implementations, a display system, a keyboard, and a pointing device may be included as part of a user interface subsystem 609 to provide for a user to manually input information. Ports for inputting and outputting data also may be included. More elements such as network connections, interfaces to various devices, and so forth, may be included, but are not illustrated in Fig. 6. The memory of the memory subsystem 605 may at some time hold part or all (in either case shown as 601) of a set of instructions that when executed on the processing system 600 implement the steps of the method embodiments described herein. A bus 613 may be provided for connecting the components. Thus, while a processing system 600 such as shown in Fig. 6 is prior art, a system that includes the instructions to implement aspects of the methods for estimating a property of a neural or cardial source is not prior art, and therefore Fig. 6 is not labelled as prior art. The method or part thereof may be implemented as an algorithm and the processing system 600 may have one or more components expressing the functionality of one or more steps of the algorithm, e.g. an algorithm as shown in FIG. 3 and FIG. 4. The computer implemented invention may be programmed such that it is performed automated and/or automatically.

The present invention also includes a computer program product which provides the functionality of any of the methods according to the present invention when executed on a computing device. Such computer program product can be tangibly embodied in a carrier medium carrying machine-readable code for execution by a programmable processor. The present invention thus relates to a carrier medium carrying a computer program product that, when executed on computing means, provides instructions for executing any of the methods as described above. The term "carrier medium" refers to any medium that participates in providing instructions to a processor for execution. Such a medium may take many forms, including but not limited to, non-volatile media, and transmission media. Non-volatile media includes, for example, optical or magnetic disks, such as a storage device which is part of mass storage. Common forms of computer readable media include, a CD-ROM, a DVD, a blue ray disk, a flexible disk or floppy disk, a tape, a memory chip or cartridge or any other medium from which a computer can read. Various forms of computer readable media may be involved in carrying one or more sequences of one or more instructions to a processor for execution. The computer program product can also be transmitted via a carrier wave in a network, such as a LAN, a WAN or the Internet. Transmission media can take the form of acoustic or light waves, such as those generated during radio wave and infrared data communications. Transmission media include coaxial cables, copper wire and fibre optics, including the wires that comprise a bus within a computer.

By way of illustration, embodiments of the present invention not being limited thereto, experimental results are discussed below, indicating standard and optional features and advantages of some embodiments of the present invention. The results discussed below are on the one hand based on results using a spherical head model and on the other hand based on a realistic head model.

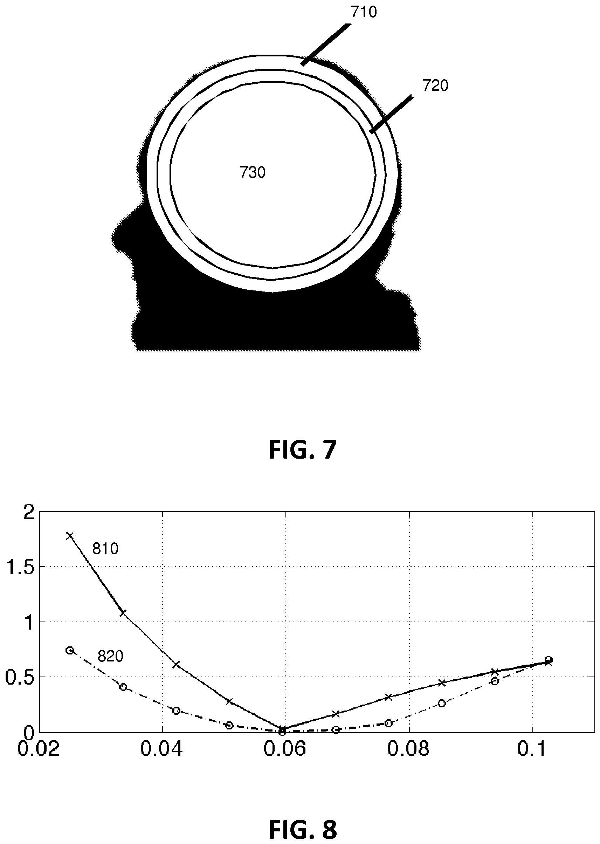

In a first set of experimental results, use is made of a spherical head model. More particularly, the channel selection methodology as described above is applied to a simplified geometry of the head, as illustrated in FIG. 7. A spherical head model 700 was used, consisting of three shells : a first shell corresponds with the scalp compartment 710 in the present example having a radius $R_3$=9.2cm, a second shell 720 corresponds with the skull compartment 720 with radius $R_2$=8.6cm and a third shell corresponds with a brain compartment 730 with radius $R_1$=8.0cm. The potentials on the surface of the head were calculated using the semi-analytical expression given in [Y. Salu, L. Cohen, D. Rose, S. Sato, C. Kufta, M. Hallett, "An improved method for localizing electric brain dipoles," IEEE Trans Biomed Eng, vol.37, pp.699-705, 1990]. The electrode potentials depend on the geometry (radius of the several shells), electrode locations and the soft tissue to skull conductivity ratio. FIG. 8 illustrates the dipole localization errors in mm (y-axis) versus differently assumed soft tissue to skull conductivity ratios (x-axis). The actual conductivity ratio value was here 0.0625. The traditional methodology 810 (indicated with crosses) and the selection methodology 820 (indicated by circles) were applied onto simulation data. The center of the head was referenced as r=[$r_x$=0, $r_y$=0, $r_z$=0] and the considered dipole here was located at [0, 0, 8.6]mm. $r_x$, $r_y$, $r_z$ are respectively the x, y and z coordinate of the dipole location.

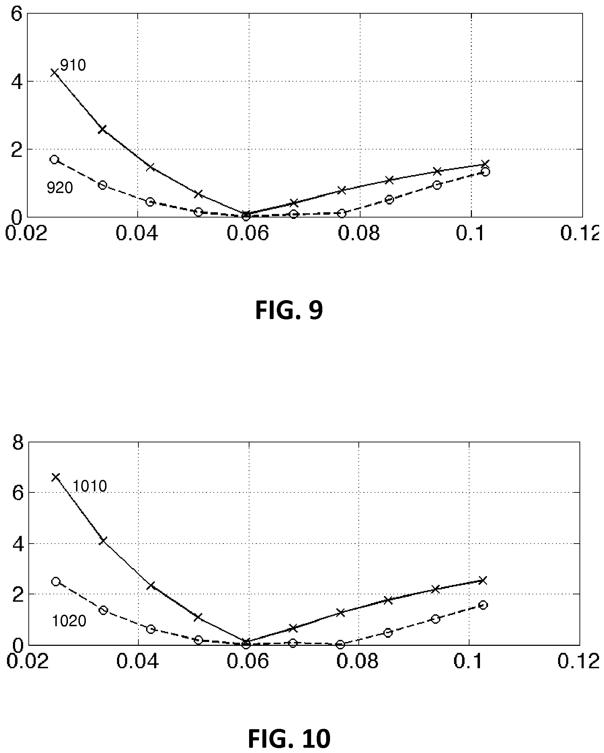

FIG. 9 depicts the dipole localization errors in mm (y-axis) versus differently assumed soft tissue to skull conductivity ratios (x-axis). The actual conductivity ratio value was here 0.0625. The traditional methodology 910 (indicated with crosses) and the selection methodology 920 (indicated with circles) were applied onto simulation data. The center of the head was referenced as r=[$r_x$=0, $r_y$=0, $r_z$=0] and the considered dipole here was located at [8.6, 17.2, 8.6]mm. $r_x$, $r_y$, $r_z$ were respectively the x, y and z coordinate of the dipole location.

FIG. 10 depicts the dipole localization errors in mm (y-axis) versus differently assumed soft tissue to skull conductivity ratios (x-axis). The actual conductivity ratio value was here 0.0625. The traditional methodology 1010 (indicated with crosses) and the selection methodology 1020 (indicated with circles) were applied onto simulation data. The center of the head was referenced as r=[$r_x$=0, $r_y$=0, $r_z$=0] and the considered dipole here was located at [17.2, 25.8, 17.2]mm. $r_x$, $r_y$, $r_z$ are respectively the x, y and z coordinate of the dipole location.

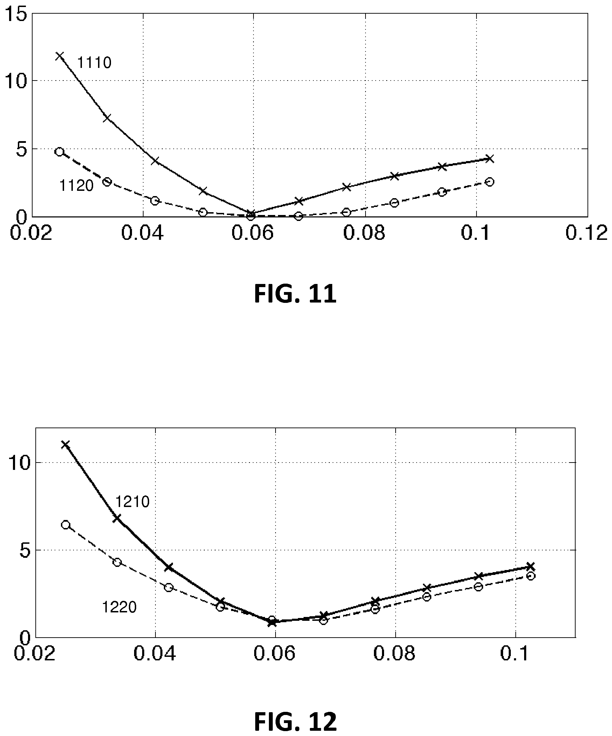

FIG. 11 depicts the dipole localization errors in mm (y-axis) versus differently assumed soft tissue to skull conductivity ratios (x-axis). The actual conductivity ratio value was here 0.0625. The traditional methodology 1110 (indicated with crosses) and the selection methodology 1120 (indicated with circles) were applied onto simulation data. The center of the head was referenced as r=[$r_x$=0, $r_y$=0, $r_z$=0] and the considered dipole here was located at [34.4, 25.8, 34.4] mm. $r_x$, $r_y$, $r_z$ are respectively the x, y and z coordinate of the dipole location.

FIG. 12 depicts the dipole localization errors in mm (y-axis) versus differently assumed soft tissue to skull conductivity ratios (x-axis). The actual conductivity ratio value was here 0.0625. The dipole was located near [34.4, 25.8, 34.4]mm with actual conductivity ratio of 0.0625. Synthetic noise data was added to the synthetic data. Graph 1210 (indicated with crosses) represents the traditional methodology, graph 1220 (indicated with circles) represents the selection methodology.

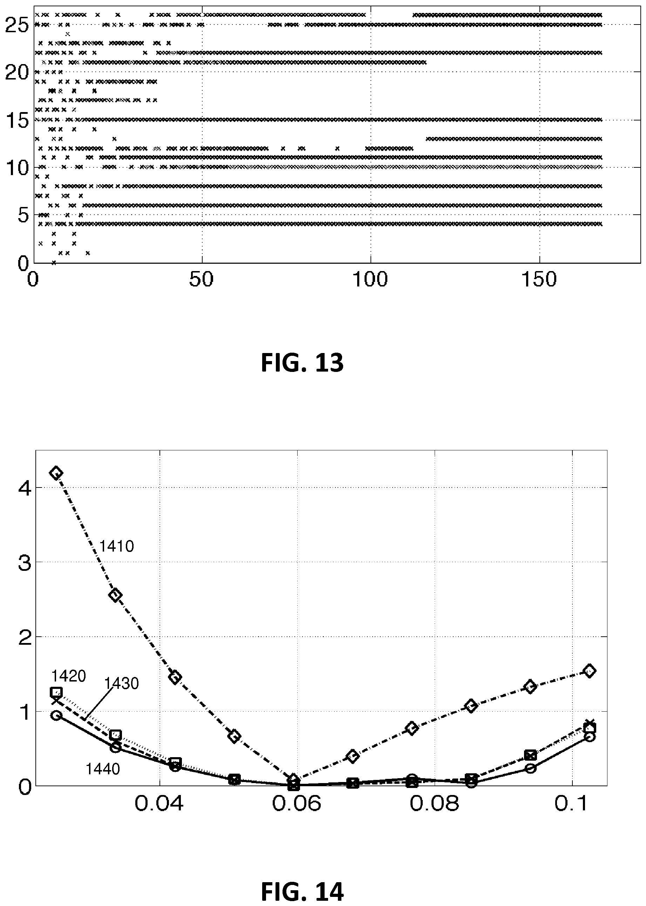

FIG. 13 depicts the employed indices (y-axis) of the selected channels in each iteration (x-axis) of the minimization procedure. A fixed number of channels (10 channels) were selected out of the total amount of 27 channels. Indices (0 till 26) are related to the specific channels used: each index refers to a specific location of the measurement channel.

FIG. 14 indicates the source localization error in mm (y-axis) versus different assumed soft tissue to skull conductivity ratios (x-axis). The actual conductivity ratio was here 0.0625. A dipole near the middle of the brain was to be recovered. The total number of channels was 112 and the figure depicts dipole position errors when using the traditional method 1410 (indicated by diamonds), the selection methodology 1420 with the number of selected channels 20 (indicated by squares), the selection methodology 1430 with the selected number of 30 channels (indicated by crosses), the selection methodology 1440 with the selected number of 40 selected channels (indicated by circles).

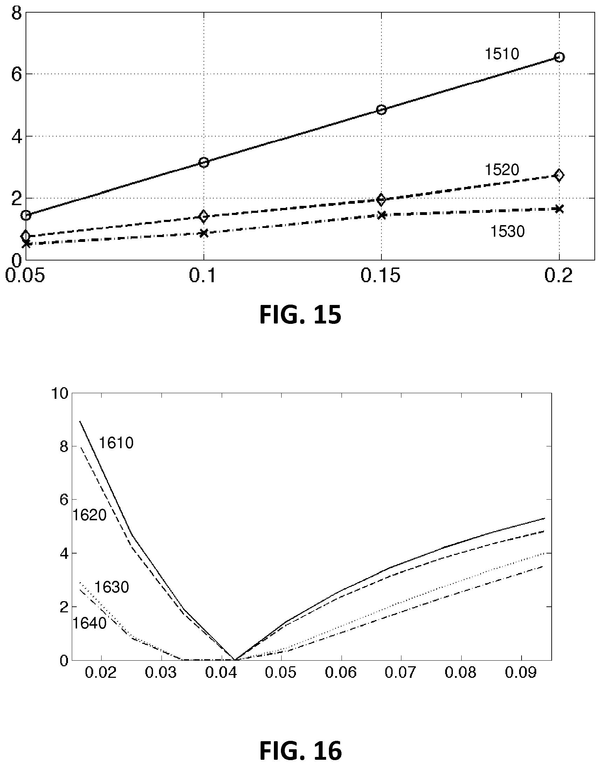

FIG. 15 shows the dipole position error in mm (y-axis) versus noise level (x-axis) using selection methodology for different hardware setups: graph 1510 (circles) illustrates the result for a EEG cap consisting of 27 channels, graph 1520 (diamonds) illustrates the result for an EEG cap consisting of 112 channels, graph 1530 (crosses) illustrates the results for an EEG cap consisting of 148 channels. The assumed conductivity ratio was here different from the actual conductivity ratio.

FIG. 16 illustrates the dipole position error in mm (y-axis) versus the assumed conductivity ratio (x-axis) for two dipoles located at [17.2, 34.4, 25.8]mm and [25.8, 43.0, 8.6]mm. Using traditional methodology, respectively dipole position errors 1610 and 1620 are observed, while using the selection methodology, dipole position errors 1630 and 1640 are observed.

The above results illustrate that the channel selection method results in a decrease of the position localization error of a neural source, the latter being illustrated for different positions of the neural source and for different conditions.

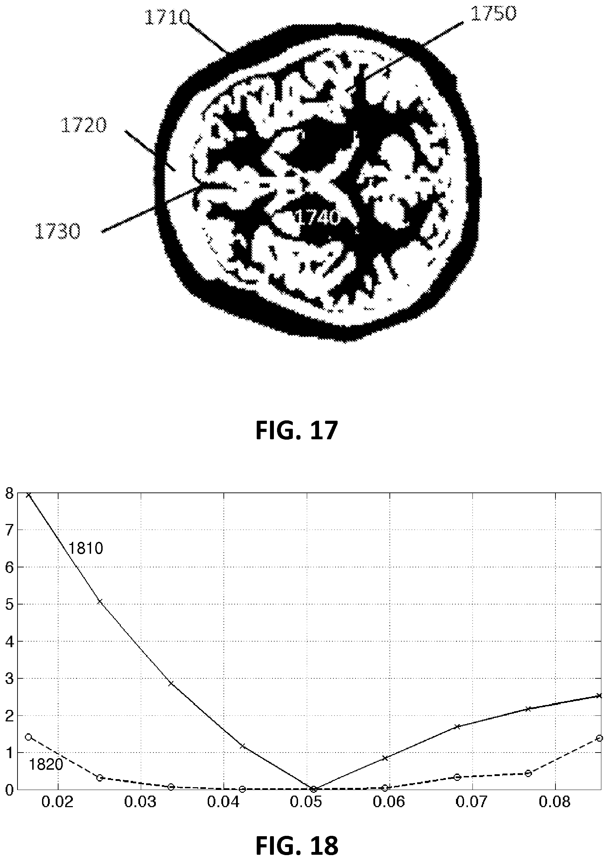

In a second set of experimental results, use is made of a more realistic head model. FIG. 17 illustrates an axial slice of the used realistic head model geometry based on T1-segmented MR images with segmentation in 5 compartments: a first compartment being the scalp 1710, a second being the skull 1720, a third being the cerebrospinal fluid 1730, a fourth being white matter 1740 and a fifth being grey matter 1750. Computations of forward EEG potentials were carried out here using finite difference method. Here, the scalp, the cerebrospinal fluid, the white matter and the grey matter had the same soft tissue conductivity while the skull had the skull conductivity. The computations of the forward EEG model depend on the soft tissue conductivity to skull conductivity ratio.

FIG. 18 illustrates the dipole position error in mm (y-axis) versus the assumed conductivity ratio (x-axis) using synthetic data in a realistic head model. The actual conductivity ratio was 0.0508. Total number of channels was 81. Graph 1810 illustrates the errors when using traditional methodology and graph 1820 illustrates the errors when using selection methodology.

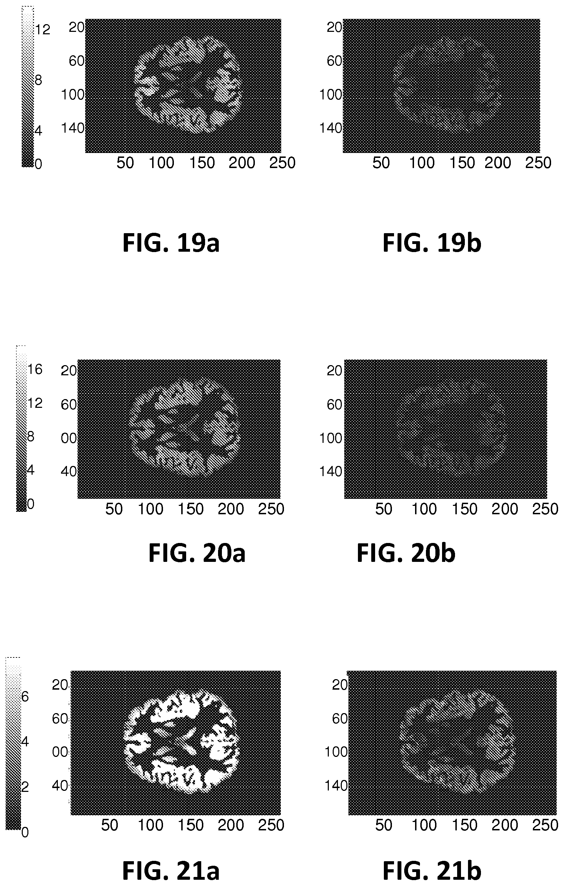

FIG. 19a to FIG. 21b illustrate dipole localization errors due to using wrong conductivity ratio when using traditional methodology (FIG. 19a, FIG. 20a, FIG. 21a) respectively selection methodology (FIG. 19b, FIG. 20b, FIG. 21b), whereby the dipoles were oriented in the x-direction (FIG. 19a, FIG. 19b), in the y-direction (FIG. 20a, FIG. 20b) and in the z-direction (FIG. 21a, FIG. 21b) respectively. It can be seen that the dipole localization errors are substantially smaller using the selection methodology compared to the traditional methodology.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

It should be noted that the use of particular terminology when describing certain features or aspects of the invention should not be taken to imply that the terminology is being re-defined herein to be restricted to include any specific characteristics of the features or aspects of the invention with which that terminology is associated.

## Claims

1. A system for estimating a property of a neural or cardial source in a subject, using inverse problem solving including a forward numerical model, the forward numerical model being used for simulating electrode potentials using lead field calculations, the model being based on geometric information of the subject, the model being based on an estimated initial property of the neural or cardial source and the model being based on a conductivity of a modeled tissue of the subject, the property being a location, an orientation, an amplitude or a dynamic behavior of an electrical activity of the neural or cardial source, the system comprising a receiving means (510) adapted for receiving a plurality of measured measurement channel results from a part of the body of the subject, the measured measurement channel results being a plurality of signals responsive to the electrical activity of the neural or cardial source, a selection means (520) adapted for selecting at least one subset of a plurality of measurement channels, said selecting comprising selecting measurement channels having a sensitivity to uncertainty in conductivity less than a predetermined value, where the sensitivity of a measurement channel being a measure of how simulated measurement channel results obtained by the forward numerical model change due to a change of uncertainty in conductivity in the forward numerical model, and a calculation means (530) adapted for determining the property of the neural or cardial source based on the selected subset of measurement channels, the calculation means comprising: a) a forward modeling means (532) adapted for obtaining simulated measurement channel results for the selected subset of measurement channels by the forward numerical model based on an estimated property of the neural or cardial source; b) a comparator (534) adapted for comparing the obtained simulated measurement channel results and the measured measurement channel results of said at least one selected subset of measurement channels; c) a property calculator (536) adapted for calculating an updated property estimate of the neural or cardial source if the measured and simulated measurement channel results differ by more than a predetermined value; and a controller (540) adapted for controlling the receiving, selection and calculation means in an iterative manner with the updated property estimate until a sufficiently accurate agreement between the simulated and measured measurement channel results is obtained.

2. A system according to claim 1, wherein the calculation means (530) is adapted for determining an estimated location of the neural or cardial source.

3. A system according to any of the previous claims, wherein the controller is adapted for selecting a new subset of measurement channels in each step of the iteration.

4. A system according to any of the previous claims, wherein said selection means (520) is adapted for furthermore taking into account the sensitivity of the simulated measurement channel results of the measurement channels to a further uncertainty in the forward numerical model for the neural or cardial source.

5. A computer-implemented method for estimating a property of a neural or cardial source in a subject, using inverse problem solving including a forward numerical model, the forward numerical model being used for simulating electrode potentials using lead field calculations, the model being based on geometric information of the subject, the model being based on an estimated initial property of the neural or cardial source and the model being based on a conductivity of a modeled tissue of the subject, the property being a location, an orientation, an amplitude or a dynamic behavior of an electrical activity of a neural or cardial source, the method comprising

- receiving a plurality of measured measurement channel results from a part of the body of the subject, the measured measurement channel results being a plurality of signals responsive to the electrical activity of the neural or cardial source;
- selecting at least one subset of a plurality of measurement channels, said selecting comprising selecting measurement channels having a sensitivity to uncertainty in conductivity less than a predetermined value, the sensitivity of a measurement channel being a measure of how simulated measurement channel results obtained by the forward numerical model change due to a change of uncertainty in conductivity in the forward numerical model, and
- estimating the property of the neural or cardial source based on the selected subset of measurement channels, the estimating comprising: a) forward modeling of the simulated measurement channel results for the subset of measurement channels based on an estimated property of the neural or cardial source; b) comparing the simulated measurement channel results and the measured measurement channel results of said at least one selected subset of measurement channels; c) calculating an updated property estimate of the neural or cardial source if the measured and simulated channel results differ by more than a predetermined value; and repeating the receiving, selecting and calculating in an interative manner with the updated property estimate until a sufficiently accurate agreement between the simulated and measured measurement channel results is obtained.

6. A method according to claim 5, wherein the estimating the property comprises estimating the location of the neural or cardial source.

7. A machine readable data storage device storing a computer program product for performing, when executed on a computer, a method according to claims 5 to 6.

**Patentansprüche**

1. System zum Bewerten einer Eigenschaft einer neuralen oder kardialen Quelle in einem Subjekt unter Verwendung von inverser Problemlösung einschließlich eines numerischen Vorwärtsmodells, wobei das numerische Vorwärtsmodell, das zum Simulieren von Elektrodenpotenzialen verwendet wird, Leitungsfeldberechnungen verwendet, wobei das Modell auf geometrischen Informationen über das Subjekt basiert, wobei das Modell auf einer bewerteten anfänglichen Eigenschaft der neuralen oder kardialen Quelle basiert und das Modell auf einer Leitfähigkeit eines modellierten Gewebes des Subjekts basiert, wobei die Eigenschaft eine Stelle, eine Ausrichtung, eine Amplitude oder ein dynamisches Verhalten einer elektrischen Aktivität der neuralen oder kardialen Quelle ist, wobei das System ein Empfangsmittel (510) umfasst, das angepasst ist, um eine Vielzahl von gemessenen Messkanalergebnissen von einem Teil des Körpers des Subjekts zu empfangen, wobei die gemessenen Messkanalergebnisse eine Vielzahl von Signalen sind, die auf die elektrische Aktivität der neuralen oder kardialen Quelle ansprechen, ein Auswahlmittel (520), das angepasst ist, um mindestens eine Untermenge einer Vielzahl von Messkanälen auszuwählen, wobei das Auswählen ein Auswählen von Messkanälen umfasst, die eine Empfindlichkeit für Leitfähigkeitsunbestimmtheit aufweisen, die kleiner ist als ein vorbestimmter Wert, wo die Empfindlichkeit eines Messkanals ein Maß dafür ist, wie simulierte Messkanalergebnisse, die vom numerischen Vorwärtsmodell erhalten werden, sich aufgrund einer Änderung von Leitfähigkeitsunbestimmtheit im numerischen Vorwärtsmodell ändern, und ein Berechnungsmittel (530), das angepasst ist, um die Eigenschaft der neuralen oder kardialen Quelle basierend auf der ausgewählten Untermenge von Messkanälen zu bestimmen, wobei das Berechnungsmittel umfasst: a) ein Vorwärtsmodellierungsmittel (532), das angepasst ist, um simulierte Messkanalergebnisse für die ausgewählte Untermenge von Messkanälen vom numerischen Vorwärtsmodell basierend auf einer bewerteten Eigenschaft der neuralen oder kardialen Quelle zu erhalten; b) einen Vergleicher (534), der angepasst ist, um die erhaltenen simulierten Messkanalergebnisse und die gemessenen Messkanalergebnisse der mindestens einen ausgewählten Untermenge von Messkanälen zu vergleichen; c) einen Eigenschaftsberechner (536), der angepasst ist, um eine aktualisierte Eigenschaftsbewertung der neuralen oder kardialen Quelle zu berechnen, falls die gemessenen und simulierten Messkanalergebnisse sich um mehr als einen vorbestimmten Wert unterscheiden; und eine Steuerung (540), die angepasst ist, um das Empfangs-, Auswahl- und Berechnungsmittel in einer iterativen Weise mit der aktualisierten Eigenschaftsbewertung zu steuern, bis eine hinreichend genaue Übereinstimmung zwischen den simulierten und gemessenen Messkanalergebnissen erhalten wird.

2. System nach Anspruch 1, wobei das Berechnungsmittel (530) angepasst ist, um eine bewertete Stelle der neuralen oder kardialen Quelle zu bestimmen.

3. System nach einem der vorstehenden Ansprüche, wobei die Steuerung angepasst ist, um bei jedem Schritt der Iteration eine neue Untergruppe von Messkanälen auszuwählen.

4. System nach einem der vorstehenden Ansprüche, wobei das Auswahlmittel (520) angepasst ist, um ferner die Empfindlichkeit der simulierten Messkanalergebnisse des Messkanals auf eine weitere Unbestimmtheit im numerischen Vorwärtsmodell für die neurale oder kardiale Quelle zu berücksichtigen.

5. Computerimplementiertes Verfahren zum Bewerten einer Eigenschaft einer neuralen oder kardialen Quelle in einem Subjekt unter Verwendung von inverser Problemlösung einschließlich eines numerischen Vorwärtsmodells, wobei das numerische Vorwärtsmodell, das zum Simulieren von Elektrodenpotenzialen verwendet wird, Leitungsfeldberechnungen verwendet, wobei das Modell auf geometrischen Informationen über das Subjekt basiert, wobei das Modell auf einer bewerteten anfänglichen Eigenschaft der neuralen oder kardialen Quelle basiert und das Modell auf einer Leitfähigkeit eines modellierten Gewebes des Subjekts basiert, wobei die Eigenschaft eine Stelle, eine Ausrichtung, eine Amplitude oder ein dynamisches Verhalten einer elektrischen Aktivität einer neuralen oder kardialen Quelle ist, wobei Verfahren umfasst

   - Empfangen einer Vielzahl von gemessenen Messkanalergebnissen von einem Teil des Körpers des Subjekts, wobei die gemessenen Messkanalergebnisse eine Vielzahl von Signalen sind, die auf die elektrische Aktivität der neuralen oder kardialen Quelle ansprechen;
- Auswählen mindestens einer Untermenge einer Vielzahl von Messkanälen, wobei das Auswählen ein Auswählen von Messkanälen umfasst, die eine Empfindlichkeit für Leitfähigkeitsunbestimmtheit aufweisen, die kleiner ist als ein vorbestimmter Wert, wobei die Empfindlichkeit eines Messkanals ein Maß dafür ist, wie simulierte Messkanalergebnisse, die vom numerischen Vorwärtsmodell erhalten werden, sich aufgrund einer Änderung von Leitfähigkeitsunbestimmtheit im numerischen Vorwärtsmodell ändern, und
- Bewerten der Eigenschaft der neuralen oder kardialen Quelle basierend auf der ausgewählten Untermenge von Messkanälen, wobei das Bewerten umfasst: a) Vorwärtsmodellieren der simulierten Messkanalergebnisse für die Untermenge von Messkanälen basierend auf einer bewerteten Eigenschaft der neuralen oder kardialen Quelle; b) Vergleichen der simulierten Messkanalergebnisse und der gemessenen Messkanalergebnisse der mindestens einen ausgewählten Untermenge von Messkanälen; c) Berechnen einer aktualisierten Eigenschaftsbewertung der neuralen oder kardialen Quelle wenn die gemessenen und simulierten Messkanalergebnisse unterscheiden sich um mehr als einen vorbestimmten Wert; und Wiederholen des Empfangens, Auswählens und Berechnens in einer iterativen Weise mit der aktualisierten Eigenschaftsbewertung, bis eine hinreichend genaue Übereinstimmung zwischen den simulierten und gemessenen Messkanalergebnissen erhalten wird.

6. Verfahren nach Anspruch 5, wobei das Bewerten der Eigenschaft ein Bewerten der Stelle der neuralen oder kardialen Quelle umfasst.

7. Maschinenlesbare Datenspeichervorrichtung, die ein Computerprogrammprodukt zum Durchführen, bei Ausführung auf einem Computer, eines Verfahrens nach Ansprüche 5 bis 6 speichert.

**Revendications**

1. Système d'estimation d'une propriété d'une source neurale ou cardiale chez un sujet en utilisant une résolution de problème inverse comprenant un modèle numérique direct, le modèle numérique direct étant utilisé pour simuler des potentiels d'électrodes en utilisant des calculs de champs conducteurs, le modèle étant basé sur des informations géométriques du sujet, le modèle étant basé sur une propriété initiale estimée de la source neurale ou cardiale et le modèle étant basé sur une conductivité d'un tissu modélisé du sujet, la propriété étant un emplacement, une orientation, une amplitude ou un comportement dynamique d'une activité électrique de la source neurale ou cardiale, le système comprenant un moyen de réception (510) qui est à même de recevoir une pluralité de résultats de canaux de mesure mesurés d'une partie du corps du sujet, les résultats de canaux de mesure mesurés étant une pluralité de signaux sensibles à l'activité électrique de la source neurale ou cardiale, un moyen de sélection (520) qui est à même de sélectionner au moins un sous-ensemble d'une pluralité de canaux de mesure, ladite sélection comprenant la sélection de canaux de mesure ayant une sensibilité à une incertitude de conductivité inférieure à une valeur prédéterminée, où la sensibilité d'un canal de mesure est une mesure de la manière dont des résultats de canaux de mesure simulés obtenus par le modèle numérique direct changent en raison d'un changement d'incertitude de conductivité dans le modèle numérique direct, et un moyen de calcul (530) qui est à même de déterminer la propriété de la source neurale ou cardiale sur la base du sous-ensemble sélectionné de canaux de mesure, le moyen de calcul comprenant a) un moyen de modélisation direct (532) qui est à même d'obtenir des résultats de canaux de mesure simulés pour le sous-ensemble sélectionné de canaux de mesure par le modèle numérique direct sur la base d'une propriété estimée de la source neurale ou cardiale ; b) un comparateur (534) qui est à même de comparer les résultats de canaux de mesure simulés obtenus et les résultats de canaux de mesure mesurés dudit au moins un sous-ensemble sélectionné de canaux de mesure ; c) un calculateur de propriété (536) qui est à même de calculer une estimation de propriété mise à jour de la source neurale ou cardiale si les résultats de canaux de mesure mesurés et simulés diffèrent de plus d'une valeur prédéterminée ; et un dispositif de commande (540) qui est à même de commander les moyens de réception, de sélection et de calcul de manière itérative avec l'estimation de propriété mise à jour jusqu'à ce qu'un accord suffisamment précis entre les résultats de canaux de mesure simulés et mesurés soit obtenu.

2. Système selon la revendication 1, dans lequel le moyen de calcul (530) est à même de déterminer un emplacement estimé de la source neurale ou cardiale.

3. Système selon l'une quelconque des revendications précédentes, dans lequel le dispositif de commande est à même de sélectionner un nouveau sous-ensemble de canaux de mesure à chaque étape de l'itération.

4. Système selon l'une quelconque des revendications précédentes, dans lequel ledit moyen de sélection (520) est à même de prendre encore en compte la sensibilité des résultats de canaux de mesure simulés des canaux de mesure à une autre incertitude dans le modèle numérique direct pour la source neurale ou cardiale.

5. Procédé mis en oeuvre par ordinateur pour estimer une propriété d'une source neurale ou cardiale chez un sujet en utilisant une résolution de problème inverse comprenant un modèle numérique direct, le modèle numérique direct étant utilisé pour simuler des potentiels d'électrodes en utilisant des calculs de champs conducteurs, le modèle étant basé sur des informations géométriques du sujet, le modèle étant basé sur une propriété initiale estimée de la source neurale ou cardiale et le modèle étant basé sur une conductivité d'un tissu modélisé du sujet, la propriété étant un emplacement, une orientation, une amplitude ou un comportement dynamique d'une activité électrique d'une source neurale ou cardiale, le procédé comprenant :

   - la réception d'une pluralité de résultats de canaux de mesure mesurés d'une partie du corps du sujet, les résultats de canaux de mesure mesurés étant une pluralité de signaux sensibles à l'activité électrique de la source neurale ou cardiale ;
   - la sélection d'au moins un sous-ensemble d'une pluralité de canaux de mesure, ladite sélection comprenant la sélection de canaux de mesure ayant une sensibilité à l'incertitude de conductivité inférieure à une valeur prédéterminée, la sensibilité d'un canal de mesure étant une mesure de la manière dont les résultats de canaux de mesure simulés obtenus par le modèle numérique direct changent en raison d'un changement d'incertitude de conductivité dans le modèle numérique direct et
   - l'estimation de la propriété de la source neurale ou cardiale sur la base du sous-ensemble sélectionné de canaux de mesure, l'estimation comprenant a) une modélisation direct des résultats de canaux de mesure simulés pour le sous-ensemble de canaux de mesure basée sur une propriété estimée de la source neurale ou cardiale ; b) la comparaison des résultats de canaux de mesure simulés et des résultats de canaux de mesure mesurés dudit au moins un sous-ensemble sélectionné de canaux de mesure ; c) le calcul d'une estimation de propriété mise à jour de la source neurale ou cardiale si les résultats de canaux mesurés et simulés différent de plus d'une valeur prédéterminée ; et la répétition de la réception, de la sélection et du calcul de manière itérative avec l'estimation de propriété mise à jour jusqu'à ce qu'un accord suffisamment précis entre les résultats de canaux de mesure simulés et mesurés soit obtenu.

6. Procédé selon la revendication 5, dans lequel l'estimation de la propriété comprend l'estimation de l'emplacement de la source neurale ou cardiale.

7. Dispositif de stockage de données lisible sur ordinateur stockant un produit de programme d'ordinateur pour effectuer, lorsqu'il est exécuté sur un ordinateur, un procédé selon les revendications 5 à 6.

**FIG. 1**

**FIG. 2**

**FIG. 3**

**FIG. 4**

**FIG. 5**

**FIG. 6**

**FIG. 7**

**FIG. 8**

**FIG. 9**

**FIG. 10**

**FIG. 11**

**FIG. 12**

**FIG. 13**

**FIG. 14**

**FIG. 15**

**FIG. 16**

**FIG. 17**

**FIG. 18**

**FIG. 19a**

**FIG. 19b**

**FIG. 20a**

**FIG. 20b**

**FIG. 21a**

**FIG. 21b**

**EP 2 499 585 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006060727 A

- US 20080161714 A1

**Non-patent literature cited in the description**

- **OOSTENDORP et al.** *IEEE Transactions on Biomedical Engineering,* 2000, vol. 47 (11), 1487-1492
- **GONCALVES et al.** *IEEE Transactions on Biomedical Engineering,* 2003, vol. 50 (6), 754-767
- **COLE et al.** *Journal of Chemical Physics,* 1941, vol. 9, 341-351

- **GABRIEL et al.** *Physics in Medicine and Biology,* 1996, vol. 41, 2271-2293
- **MOSHER ; LEAHY.** *IEEE Transactions on Signal Processing,* 1999, vol. 47, 332-340
- **Y. SALU ; L. COHEN ; D. ROSE ; S. SATO ; C. KUFTA ; M. HALLETT.** An improved method for localizing electric brain dipoles. *IEEE Trans Biomed Eng,* 1990, vol. 37, 699-705